# EUROPÄISCHE PATENTSCHRIFT

(11) **EP 1 108 470 B1**
(45) Veröffentlichungstag und Bekanntmachung des Hinweises auf die Patenterteilung: **17.07.2002**
(21) Anmeldenummer: 00124327.8
(22) Anmeldetag: 16.11.2000
(51) Int. Cl.: B01J 35/02, C07C 51/265, C07C 51/31, C07C 51/215, C07C 51/25, C07C 53/08, C07C 63/16

(54) **Trägerkatalysatoren und deren Verwendung bei der Gasphasenoxidation von Kohlenwasserstoffen**
Supported catalyst and the use thereof for the gas phase oxidation of hydrocarbons
Catalyseur sur support et son utilisation pour l'oxydation des hydrocarbures en phase vapeur

(30) Priorität: 09.12.1999 DE 19959413
(43) Veröffentlichungstag der Anmeldung: 20.06.2001
(73) Patentinhaber: Consortium für elektrochemische Industrie GmbH, 81379 München (DE)
(72) Erfinder: Eberle, Hans-Jürgen, Dr., 81477 München (DE); Groke, Dirk, Dr., 82024 Taufkirchen (DE); Rüdinger, Christoph, Dr., 82319 Starnberg (DE); Wecker, Ulrich, Dr., 82547 Eurasburg (DE)
(74) Vertreter: Schuderer, Michael, Dr.

(56) Entgegenhaltungen:
- DE-A- 19 721 368
- GB-A- 1 373 351
- GB-A- 2 197 597
- US-A- 2 408 164
- US-A- 4 645 754

## Beschreibung

Die Erfindung betrifft Trägerkatalysatoren und deren Verwendung bei der Gasphasenoxidation von Kohlenwasserstoffen.

Trägerkatalysatoren für die Gasphasenoxidation von Kohlenwasserstoffen zu den entsprechenden Oxidationsprodukten wie beispielsweise Carbonsäuren, Carbonsäureanhydriden oder Aldehyde sind seit langem bekannt. Ein typisches Anwendungsbeispiel für derartige Katalysatoren ist die Herstellung von Phthalsaureanhydrid aus o-Xylol oder Naphthalin, Maleinsaureanhydrid aus Benzol oder Butan, Formaldehyd aus Methanol, Acrylsäure oder Acrolein aus Propen. Neuerdings ist auch die Herstellung von Essigsäure durch die Oxidation von Ethan oder Butan oder Buten sowie Butan/Buten-Gemischen mit Trägerkatalysatoren beschrieben. Allen diesen Herstellungsprozessen ist gemeinsam, daß die Reaktionen stark exotherm ablaufen. Aus diesem Grund werden nahezu alle derartigen Prozesse in sogenannten Röhrenreaktoren durchgeführt. Hierbei sind die Rohre mit einem Katalysator gefüllt, die Ableitung der entstehenden Reaktionswärme (Kühlung) erfolgt üblicherweise über eine Salzschmelze, mit der die Reaktionsrohre innerhalb des Reaktors umgeben sind. Die Kühlung kann in Abhängigkeit des Temperaturbereichs alternativ auch mit Dampf, überhitztem Wasser oder anderen Warmeträgerflussigkeiten erfolgen.

Bei den verwendeten Katalysatoren handelt es sich überwiegend um Trägerkatalysatoren, die in der Regel aus einem inerten Trägerkörper, beispielsweise in Ring- oder Kugelform, bestehen, auf dem die eigentlich katalytisch aktive Masse aufgebracht ist. Derartige katalytisch aktive Massen bestehen beispielsweise bei den PSA- und Essigsäurekatalysatoren überwiegend aus TiO₂ in Anatasform und V₂O₅. Zur Verbesserung der Steuerung der Aktivität und Verbesserung der Selektivität werden dabei häufig zusätzlich noch aktivierende oder auch dämpfende Zusätze, beispielsweise Oxide von Elementen der Nebengruppen des Periodensystems, Alkaliverbindungen, und/oder in geringen Mengen Promotoren als Dotierstoffe, der katalytisch aktiven Masse beigemischt. Bei Katalysatoren für die Herstellung von MSA besteht die katalytisch aktive Masse beispielsweise aus Vanadylpyrophosphat.

Bei der Herstellung der Trägerkatalysatoren werden im allgemeinen Suspensionen aus Katalysatorpulver und Flüssigkeiten (Wasser, organische Lösungsmittel) oder auch Lösungen oder Suspensionen der einzelnen Katalysatorbestandteile, gegebenenfalls unter Zugabe von Bindemittel zur Haftungsverbesserung der Aktivkomponenten auf dem Träger, auf die Trägerkörper aufgesprüht.
Aus EP-B 714700 (US-A 5,677,261) ist auch der Auftrag von trockenem Pulver auf angefeuchtete Trägerkörper bekannt.

Als Trägerkörper werden üblicherweise regelmäßig geformte, mechanisch stabile Körper wie Kugeln, Ringe, Halbringe, Sättel verwendet. Die Größe der Trägerkörper wird dabei vorwiegend von der Dimension des Reaktors, vor allem vom Innendurchmesser der einzelnen Reaktionsrohre bestimmt. Als Trägermaterial finden dabei beispielsweise Steatit, Duranit®, Steingut, Siliciumdioxid, Siliciumcarbid, Aluminate, Metalle, und Metallegierungen Verwendung.

Bei der Wahl der Trägerform und dessen Dimension spielt insbesondere der damit verbundene Druckverlust eine wichtige Rolle. Ein geringer Druckverlust der Katalysatorschüttung kann eine erhebliche Energieeinsparung, beispielsweise bei der Gebläseenergie, bedeuten.
Ein weiteres Kriterium besteht darin, daß die Trägermaterialen möglichst kostengünstig produziert werden können. In der Technik haben sich dabei Ringe und Kugeln durchgesetzt, wobei aufgrund des bei den Ringen vorhandenen geringeren Druckverlustes ringförmige Träger immer mehr Anwendung finden.

In der Vergangenheit hat es nicht an Versuchen gefehlt, durch Variation der Ringform Trägermaterialen zu finden, die ein Optimum an Druckverlust (d.h. einen möglichst niedrigen Druckverlust) aufweisen und dabei möglichst viel aktive Masse tragen, ohne aber die anderen Leistungsdaten wie Selektivität, Stabilität, Produktivität etc. zu mindern.

DE-A 3445289 (US-A 4,656,157) beschreibt beispielsweise einen Ringtrager, der sich von "normalen Ringen" dadurch unterscheidet, daß seine Stirnflächen abgerundet sind. Dieser Ringträger soll eine gleichmäßigere Füllung der Reaktionsrohre und damit eine gleichmäßigeren Reaktionsablauf ermöglichen. Bezüglich des Druckaufbaus wird nichts beschrieben.

Aus EP-B 552287 ist ein Vollkontakt-Katalysator zur Herstellung von Maleinsäureanhydrid beschrieben, der aus einer massiven geometrischen Form besteht, bei der in dessen äußerer Oberfläche mindestens ein Hohlraum angeordnet ist. Dabei werden in den Beispielen ausschließlich Formen beschrieben, bei denen die Hohlräume an den äußeren Oberflächen und nicht an den Stirnseiten angeordnet sind. Ziel dieser Formen ist es, eine möglicht große Oberfläche der Vollkontakt-Katalysatoren zu erhalten. Die gezeigten Formen sind dabei technisch nur mit großem Aufwand und hohen Kosten zu realisieren.

Aus EP-A 220933 ist ein Vollkontakt-Katalysator zur Verwendung in katalytischen Prozessen bekannt, der eine "vier-flügelige" Form aufweist. Diese Form wird dabei durch extrudieren der Katalysatormasse erreicht. Durch seine spezielle Form besitzt der Katalysator bessere physikalische Eigenschaften bezüglich Bruchfestigkeit und Druckaufbau.

GB-A 2193907 beschreibt einen Katalysator in zylindrischer Form, dessen Außenhülle in längsrichtung mit Rippen versehen ist, die derart dimensioniert und angeordnet sind, daß sich die einzelnen Katalystorkörper nicht verhaken können.

US-A 4,328,130 beschreibt ebenfalls eine Katalysatorform, bei der ein Zylinder in längsrichtung mit mehreren Kanälen und Rippen durchzogen ist, wobei die Aussparungen schmäler als die Rippen sind, um ein Verhaken zu vermeiden.

Aus EP-A 004079 (US-A 4,370,492 und US-A 4,370,261) sind Katalysatorformen bekannt, bei denen es sich um Strangabschnitte mit sternförmigen Querschnitt oder um gerippte Stränge handelt.

US-A 3,966,644 beschreibt Katalysatorformen aus extrudierten Strangpresslingen in Form von mehreren parallel aneinander gefügten Zylindern.

Die im Stand der Technik beschriebenen Formen für Katalysatorträger mit verringertem Druckaufbau weisen alle sehr komplizierte Formen auf. Die Herstellung ist dabei meist mit hohen Kosten verbunden und damit im großtechnischen Rahmen unwirtschaftlich.
Viele dieser komplizierten Formen, die vor allem durch extrudieren erhältlich sind, sind auf Grund Ihrer Oberfläche nicht zum Beschichten mittels aktiver Katalysatormasse geeigent, und können somit nur als Vollkontakt verwendet werden.

Ziel war es deshalb Katalysatorträger bereitzustellen, die zum einen weniger Druckaufbau als herkömmliche Ringe oder Kugeln im Reaktor besitzen, aber zum anderen eine möglichst einfache aber hochgeometrische und damit beschichtbare Oberfläche aufweisen. Zudem sollten diese Trägerformen einfach und kostengünstig herstellbar sein und in der Geometrie nur unwesentlich von den industriell verwendeten Trägern abweichen, um diese problemlos in vorhandenen Oxidationsanlagen und Prozessen verwenden zu können. Weiterhin sollten die zu entwickelnden Trägerkatalysatoren eine ebenso gute Stabilität wie die aus dem Stand der Technik bekannten aufweisen, mit den bekannten Füllmaschinen in die Reaktionsrohre einzubringen sein und beim Beschichten mit aktiver Masse eine gleichmäßige Schichtdicke ermöglichen.

Gegenstand der Erfindung sind Trägerkatalysatoren, bestehend aus einer aktiven Masse auf inerten Trägerkörpern in Form von Hohlzylindern, dadurch gekennzeichnet, daß die Hohlzylinder eine oder mehrere Kerben an der oberen und/oder unteren Flachseite der Hohlzylinderwände besitzen, so daß der Innenraum der Hohlzylinder mit den die Hohlzylinder umgebenden Räumen über Öffnungen verbunden ist.

Die Anzahl der Kerben auf den Flachseiten des erfindungsgemäßen Katalysators richtet sich nach den Anforderungen der entsprechenden Reaktion und der Dimension der Ringträger. Mindestens eine Flachseite des Ringes ist dabei mit mindestens einer Kerbe versehen. Bevorzugt sind Trägerkörper, bei denen jede der beiden Flachseiten des Ringes mit einer oder mehreren Kerben ausgestattet sind. Bevorzugt werden Katalysatoren mit 2 bis 8 Kerben auf jeder Flachseite, besonders bevorzugt 2 bis 4 Kerben.

Die Größe der verwendeten Ringe richtet sich in erster Linie nach den Anforderungen, d.h. der Größe des Reaktors. Der Trägerdurchmesser sollte dann zwischen 1/2 und 1/10 des Innendurchmessers der Reaktionsrohre betragen, bevorzugt zwischen 1/3 und 1/5. Als Materialien eignen sich beispielsweise Steatit, Duranit®, Siliciumcarbid, Steingut, Porzellan, Siliciumdioxid, Silicate, Aluminiumoxid, Aluminate oder Mischungen aus diesen Stoffen. Diese können dicht gesintert, oder auch von poröser Struktur sein. Vorzugsweise werden Ringe aus Steatit (dichtgesintertes Magnesiumsilicat) mit einer Höhe von 4 bis 10 mm, einem Außendurchmesser von 6 bis 10 mm und einer Wandstärke von 1 bis 2 mm eingesetzt.

Die erfindungsgemäßen Kerben auf den jeweiligen Seiten können regelmäßig oder unregelmäßig verteilt vorliegen. Die Anordnung der Kerben auf den Flachseiten der Ringe wird dabei bevorzugt so gewählt, daß die Kerben der gegenüberliegenden Seiten immer "auf Lücke" stehen. Beispielsweise sind somit bei einem erfindungsgemäßen Katalysator mit jeweils zwei Kerben auf jeder Flachseite die Kerben auf der gegenüberliegenden Flachseite um 90 Grad versetzt angeordnet. Die Form der Kerben kann halbrund, rechteckig, trapezförmig oder V-förmig sein.

Bei der Ausgestaltung der Kerben ist es dabei vorteilhaft, wenn die einzelnen Kerben etwas kleiner oder deutlich größer sind als die Ringdicke. Auf diese Weise können Verhakungen vermieden werden, die unter anderem den Beschichtungsprozeß erschweren. Die Tiefe und die Breite der Kerben wird dabei durch die mechanische Stabilität des Trägers bestimmt. Die Dimension der Kerben muß mindestens so groß sein, daß die Kerben beim Beschichten mit aktiver Masse nicht verschlossen werden. Die maximale Kerbentiefe und auch deren Breite werden dadurch beschränkt, daß es bei den weiteren Herstellungsschritten wie auch Anwendungsschritten (Reaktorbefullung) zu keiner Zerstörung des Katalysators kommen darf.

Bevorzugt werden Kerben mit einer Tiefe von 1/3 bis 1/2 Höhe des Ringkorpers, wenn oben und unten Kerben angebracht sind. Ist nur eine Seite des Katalysators mit den erfindungsgemäßen Kerben versehen, so kann die Tiefe der Kerben auch großer als die halbe Tiefe des Ringkörpers sein. Letztendlich ist die maximale Kerbentiefe von der verbleibenden Stabilität des Trägers abhangig.

Durch das Anbringen von je zwei Kerben in der oberen und unteren Stirnseite des Ringes lassen sich die Druckverluste um ca. 30 % reduzieren, ohne dadurch einen nennenswerten Verlust an beschichtbarer Oberfläche zu erhalten.

Überraschenderweise konnte am Beispiel der Gasphasenoxidation von Butan, Buten und deren Gemischen zu Essigsäure gezeigt werden, daß die erfindungsgemäßen Katalysatoren neben dem Vorteil des reduzierten Druckaufbaues im Reaktor zusätzlich eine deutliche Selektivitätssteigerung bei der Umsetzung der Edukte um 4 % aufwiesen. Weiterhin konnte auch noch beobachtet werden, daß die Ausbildung von Hot-Spots in der Hauptreaktionszone deutlich milder ausfallen, wie dies das Beispiele der o-Xyloloxidation zu PSA zeigt.

Anhand der folgenden Beispiele soll die Erfindung näher erläutert werden.

### Beispiel 1: (Formbeispiele)

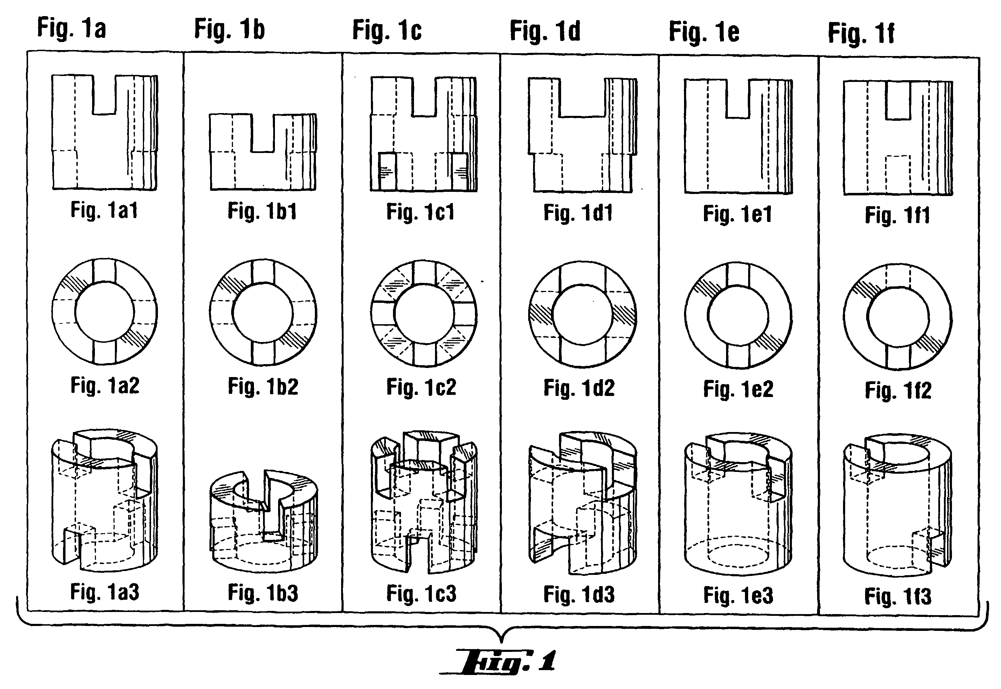

Figur 1 a-f zeigt einige Formbeispiele des erfindungsgemaßen Katalysators mit verschiedenen Anzahlen und Anordnungen der Kerben. Der Katalysatorkorper ist dabei jeweils in einer Seitenansicht (1), Draufsicht (2) und einer perspektivischen Darstellung (3) gezeigt:
a.) je zwei Kerben an der Ober- und Unterseite, 90° zueinander versetzt, Kerbentiefe 1/3 der Ringhohe, Kerbenbreite geringer als Ringdicke.
b.) je zwei Kerben an der Ober- und Unterseite, 90° zueinander versetzt, Kerbentiefe 1/2 der Ringhöhe, Kerbenbreite geringer als Ringdicke.
c.) je vier Kerben an der Ober- und Unterseite, 45° zueinander versetzt, Kerbentiefe 1/3 der Ringhohe, Kerbenbreite geringer als Ringdicke.
d.) je zwei Kerben an der Ober- und Unterseite, 90° zueinander versetzt, Kerbentiefe 1/3 der Ringhohe, Kerbenbreite größer als Ringdicke.
e.) zwei Kerben auf einer Seite, Kerbentiefe 1/3 der Ringhohe, Kerbenbreite geringer als Ringdicke.
f.) je eine Kerbe an der Ober- und Unterseite, 180° zueinander versetzt, Kerbentiefe 1/3 der Ringhohe, Kerbenbreite geringer als Ringdicke.

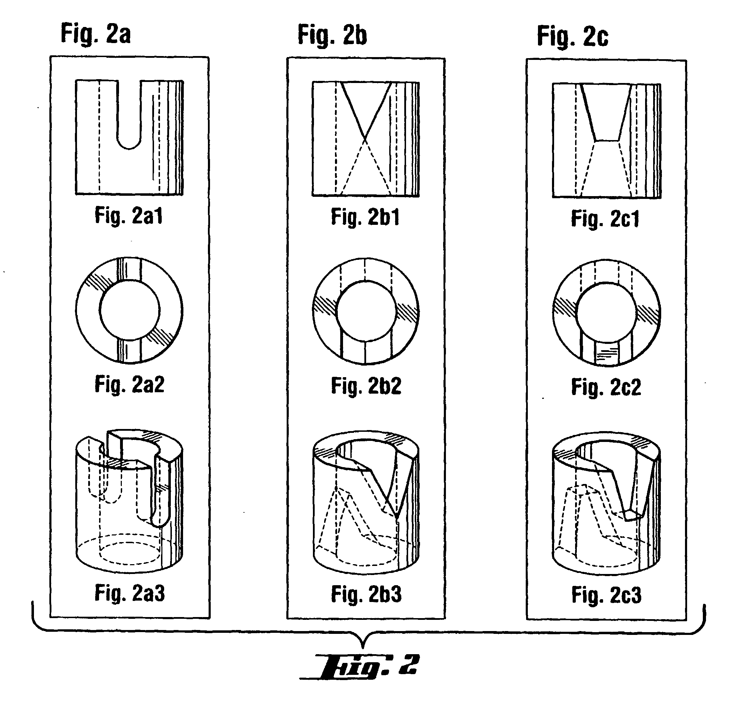

Figur 2 a-c zeigt einige Beispiele des erfindungsgemäßen Katalysators mit verschiedenen geometrischen Ausgestaltungen der Kerben. Der Katalysatorkörper ist dabei jeweils in einer Seitenansicht (1), Draufsicht (2) und einer perspektivischen Darstellung (3) gezeigt:
a.) U-formige Kerbe
b.) V-förmige Kerbe
c.) Trapezförmige Kerbe

### Beispiel 2: (Druckverlustmessungen)

Eine Meßapparatur mit einer Rohrlange von 348 cm, einem Innendurchmesser von 25 mm, einem Druckregler (0-7,5 bar), einem Rotameter (0-5 Nm³/h), einer Vordruckkammer und einem Staudruck-Manometer wurde mit Katalysatorringen befüllt. Die Fullhohe betrug 280 cm für die Ringe A, B, C und D, bzw. 72 cm für die Ringe E und F.
Zur Staudruckmessung wurde die Netzdruckluft (6 bar) mit einem Druckregler auf 2,5 bar reduziert. Am Rotameter wurden die erforderlichen Luftmengen (3 und 4 Nm³/h) mit einem Vordruck von 1,5 bar eingestellt. Die Drücke konnten dann am Staudruckmanometer abgelesen werden. Die Messung erfolgte bei Raumtemperatur.
Die Messung wurde dabei mit beschichteten (C,D) und unbeschichteten (A,B) Katalysatorringen durchgeführt. Die beschichteten Ringe waren mit jeweils 8 Gew.-% katalytischer Masse, bestehend aus V₂O₅/TiO₂ beschichtet. Zum Einsatz kamen gekerbte Ringe mit jeweils zwei Kerben an der Oberseite und zwei Kerben an der Unterseite (A,C). Die Kerben waren dabei um 90 Grad zueinander versetzt. Der Ring hatte eine Dimension von 7x7x4 mm (Außendurchmesser x Höhe x Innendurchmesser), die Kerbenbreite betrug 1,4 mm und die Kerbentiefe 2 mm. Zum Vergleich wurden Ringe mit einer Dimension von 7x7x4 mm gemessen, die nicht mit Kerben versehen waren.
Zusätzlich wurden kleinere Ringe mit einer Dimension von 7x4x4 mm gemessen (E). Diese Ringe waren auf jeder Seite mit zwei rechteckigen Kerben versehen. Die Kerben waren dabei um 90 Grad zueinander versetzt und hatten jeweils eine Tiefe von 2 mm und eine Breite von 1,4 mm. Als Vergleich hierzu wurden Ringe mit der gleichen Dimension, jedoch ohne Kerben gemessen (F).
Die Ergebnisse aus diesen Messungen sind in Tabelle 1 und Tabelle 2 dargestellt.

**Tabelle (1)**

| | | Druck (bar) bei 3 Nm³/h | Druck (bar) bei 4 Nm³/h |
|---|---|---|---|
| **A** | Gekerbte Ringe unbeschichtet 7x7x4 mm | **0,069** | **0,106** |
| **B** | Ungekerbte Ringe unbeschichtet 7x7x4 mm | **0,082** | **0,128** |
| **C** | Gekerbte Ringe beschichtet 7x7x4 mm | **0,064** | **0,099** |
| **D** | Ungekerbte Ringe beschichtet 7x7x4 mm | **0,079** | **0,120** |
| **E** | Gekerbte Ringe unbeschichtet 7x4x4 mm | **0,026** | **0,039** |
| **F** | Ungekerbte Ringe unbeschichtet 7x4x4 mm | **0,038** | **0,059** |

**Tabelle (2)**

| | Δp (bar) bei 3 Nm³/h | Δp (%) bei 3 Nm³/h | Δp (bar) bei 4 Nm³/h | Δp (%) bei 4 Nm³/h |
|---|---|---|---|---|
| **p(B-A)** | **0,012** | **18,8** | **0,022** | **20,8** |
| **p(D-C)** | **0,015** | **23,4** | **0,021** | **21,2** |
| **p(F-E)** | **0,012** | **32** | **0,02** | **34** |

Anhand der tabellarischen Auswertung der Versuchsergebnisse ist deutlich zu sehen, daß die erfindungsgemäßen Katalysatoren weniger Druckaufbau gegenüber herkömmlichen ungekerbten Katalysatoren bewirken. Erfindungsgemäße Katalysatoren mit einer Große von 7x7x4 mm besitzen dabei ca. 20 % weniger Druckaufbau, bei den kleineren Ringen mit 7x4x4 mm ist dieser Effekt sogar bis zu 34 % großer.

### Beispiel 3: (Herstellung des erfindungsgemaßen Katalysators für die Synthese von Phthalsaureanhydrid)

Zur Herstellung der Katalysatoren wurden 11,3 g V₂O₅, 70,8 g TiO₂ (BET 8m²/g), 17,7 g TiO₂ (BET 200 m²/g) und 0,2 g Cäsium (als CsCO₃) in 400 ml entionisiertem Wasser suspendiert und 18 Stunden gerührt, um eine homogene Verteilung zu erzielen. Dieser Suspension wurden 1,5 g organischer Binder, bestehend aus einem Copolymer aus Vinylacetat und Vinyllaurat in Form einer 50 Gew%-igen wäßrigen Dispersion zugegeben. Die erhaltene Suspension wurde anschließend auf 1203g Steatit-Ringe mit jeweils zwei Kerben an der Oberseite und zwei Kerben an der Unterseite aufgesprüht und getrocknet. Die Kerben waren dabei um 90 Grad zueinander versetzt. Die Ringe hatten eine Dimension von 7x7x4 mm, die Kerbenbreite betrug 1,4 mm und die Kerbentiefe 2 mm.

### Vergleichsbeispiel 1: (Katalysator aus Beispiel 3 auf ungekerbten Ringen)

Es wurde ein Katalysator analog Beispiel 3 hergestellt, jedoch mit dem Unterschied, daß er auf ungekerbten Steatit-Ringen der Große 7x7x4 mm aufgetragen wurde.

### Beispiel 4: (Herstellung eines erfindungsgemäßen Katalysators für die Synthese von Essigsaure)

Die Herstellung des Katalysators erfolgte analog DE-A-19649426. Die aktive Masse besteht aus Oxiden von Titan, Vanadium, Molybdän und Antimon der empirischen Formel TiₐV_{b}Mo_{c}Sb_{d}Oₑ (a: 91; b: 7; c: 1; d: 3; e: 207) und in einem Anteil von 14,4 Gew.-% plus 1,6 Gew.-% Graphit bezogen auf das Gewicht des Trägers. Diese aktive Masse wurde auf gekerbte Steatit-Ringe mit jeweils zwei Kerben an der Oberseite und zwei Kerben an der Unterseite aufgebracht und getrocknet. Die Kerben waren dabei um 90 Grad zueinander versetzt. Die Ringe hatten eine Dimension von 7x7x4 mm, die Kerbenbreite betrug 1,4 mm und die Kerbentiefe 2 mm.

### Vergleichsbeispiel 2: (Katalysator aus Beispiel 4 auf ungekerbten Ringen)

Die Herstellung des Katalysators erfolgte analog Beispiel 4 mit der Ausnahme, daß die aktive Masse auf ungekerbte Ringe mit einer Dimension von 7x7x4 mm (Außendurchmesser x Innendurchmesser x Hohe) aufgebracht wurde.

### Beispiel 5: (Test der Katalysatoren aus Beispiel 3 und Vergleichsbeispiel 1 am Beispiel der o-Xylol-Oxidation)

Der Test wurde in einem Rohrreaktor mit einer Länge von 330 cm und einem Innenrohrdurchmesser von 25 mm durchgeführt. Das Rohr wurde mit einem umgewälzten Salzbad (eutektische Schmelze, aus Kaliumnitrat und Natriumnitrit) temperiert. Der Reaktor wurde mit dem erfindungsgemäßen Katalysator aus Beispiel 3 bzw. dem Katalysator aus dem Vergleichsbeispiel 1 befullt. Die Katalysatorfullhöhe betrug bei beiden Versuchen 280 cm. Die Salzbadtemperaturen betrug 365°C. Nach dem Befullen wurden durch diesem Reaktor 4 Nm³ eines Luft-/o-Xylol-Gemisches durchgeleitet, wobei die o-Xylol-Konzentration 60 g/Nm³ Luft betrug und das Luft-/o-Xylol-Gemisch vor Eintritt in den Reaktor auf 180 °C vorgewärmt war. In dem Reaktor zentriert war ein Thermoelement installiert, das die Messung des Temperaturverlaufes im Rohr ermöglichte.
Das den Reaktor verlassende Reaktionsgas wurde durch einen Desublimator geleitet, um die Reaktionsprodukte wie PSA abzuscheiden.
Die Ergebnisse der Versuche sind in der folgenden Tabelle 3 dargestellt.

**Tabelle (3)**

| Ergebnisse der o-Xylol-Oxidationsversuche | | | |
|---|---|---|---|
| | Druckverlust bei 4 Nm³/h | Ausbeute an PSA in Gew-%. | Maximal-Temperatur im Katalysatorbett in °C (= Hot-spot) |
| erfindungsgemäßer Katalysator | 0,33 bar | ∼113 | 434 |
| Vergleichs-Katalysator | 0,40 bar | ∼111 | 449 |

Aus den Ergebnissen in Tabelle (3) ist ersichtlich, daß der erfindungsgemäße, gekerbte Katalysator einen deutlich geringeren Druckverlust aufweist. Die Produktausbeute ist beim erfindungsgemäßen Katalysator ebenfalls besser. Anhand des Vergleichs der maximalen Temperaturen (Hot-spot) im Reaktionsrohr ist zu erkennen, daß der erfindungsgemäße Katalysator überraschenderweise zusätzlich eine niedrigere Hot-Spot-Temperatur aufweist.

### Beispiel 6: (Test der Katalysatoren aus Beispiel 4 und Vergleichsbeispiel 2 am Beispiel der Oxidation von Butan/Butengemischen zu Essigsäure)

Der erfindungsgemäße Katalysator aus Beispiel 4 wurde in einem Kreisgasreaktor mit einem Reaktionsrohr-Innendurchmesser von 25 mm mit einer Füllhöhe von 6000 mm eingebracht, und gemäß DE-A 19910866 am Beispiel einer Oxidation eines Buten-/Butan-Gemisches getestet. Als Reaktionsgas wurden 320 g/h Sauerstoff, 130 g/h 1-Buten und 56 g/h n-Butan eingespeist. Der Kreisgasfluß wurde so eingestellt, daß der Reaktor im stabilen Zustand einen Kreisgasfluß von 12780 g/h erreichte. Der Reaktor wurde bei 11*10⁵ Pa Druck und 187°C Kühlmitteltemperatur betrieben.
Die Säureabtrennung aus dem Reaktionsgas erfolgte durch Absorption mit 1000 g/h Wasser (Aufgabe am Kopf) in einem Absorber mit strukturierter Packung, einem Innendurchmesser von 43 mm und einer Packungshöhe von 3240 mm bei einer Kopftemperatur des Absorbers von 130°C.
Unter diesen Bedingungen wurde ein Butenumsatz von 99,8 % und ein Butanumsatz von 83,1 % erreicht.

Die Essigsäureselektivität bezuglich des gesamt C₄-Umsatzes betrug 73 mol-%, die Ameisensäureselektivität bezüglich des gesamt C₄-Umsatzes betrug 9 mol-%. Die Rohsäurekonzentration betrug 24 Gew.-%.

In einem weiteren Versuch wurde analog der obigen Vorschrift mit dem Vergleichskatalysator aus Vergleichsbeispiel 2 verfahren (333 g/h Sauerstoff, 130 g/h 1-Buten und 60 g/h n-Butan). Der Kreisgasfluß wurde so eingestellt, daß der Reaktor im stabilen Zustand einen Kreisgasfluß von 12520 g/h erreichte. Der Reaktor wurde bei 11*10⁵ Pa Druck und 191°C Kühlmitteltemperatur betrieben.

Die Säureabtrennung aus dem Reaktionsgas erfolgte durch Absorption mit 1000 g/h Wasser (Aufgabe am Kopf) in einem Absorber mit strukturierter Packung, einem Innendurchmesser von 43 mm und einer Packungshöhe von 3240 mm bei einer Kopf- ° temperatur des Absorbers von 130°C.
Unter diesen Bedingungen wurde ein Butenumsatz von 99,8 % und ein Butanumsatz von 83,8 % erreicht. Die Essigsäureselektivität bezüglich des gesamt C₄-Umsatzes betrug 70 mol-%, die Ameisensäureselektivität bezüglich des gesamt C₄-Umsatzes betrug 8 mol-%. Die Rohsäurekonzentration betrug 23 Gew.-%.

Diese Ergebnisse zeigen, daß der erfindungsgemaße, gekerbte Katalysator überraschenderweise unter nahezu gleichen Versuchsbedingungen eine um 3 mol-% höhere Essigsäure-Selektivität sowie um 1 % höhere Ameisensäureselektivität ergab. Daraus ergibt sich eine um 4 % höhere Gesamtsäureselektivität dieser Reaktion. Gleichzeitig betrug der Druckverlust der Katalysatorpackung um etwa 20 % weniger als der des Vergleichskatalysators. Aufgrund dieser Tatsache ergibt sich eine enorme Energieeinsparung bei derartigen Reaktionen.

## Patentansprüche

1. Trägerkatalysatoren, bestehend aus einer aktiven Masse auf inerten Trägerkörpern in Form von Hohlzylindern, **dadurch gekennzeichnet, daß** die Hohlzylinder eine oder mehrere Kerben an der oberen und/oder unteren Flachseite der Hohlzylinderwände besitzen, so daß der Innenraum der Hohlzylinder mit den die Hohlzylinder umgebenden Räumen über Öffnungen verbunden ist.

2. Trägerkatalysatoren nach Anspruch 1, **dadurch gekennzeichnet, daß** die Anzahl der Kerben auf einer Flachseite des Hohlzylinders zwischen 2 und 8 liegt.

3. Trägerkatalysatoren nach Anspruch 1 bis 2, **dadurch gekennzeichnet, daß** die Kerben regelmäßig verteilt sind.

4. Trägerkatalysatoren nach Anspruch 1 bis 3, **dadurch gekennzeichnet, daß** die Kerben unregelmäßig verteilt sind.

5. Trägerkatalysatoren nach Anspruch 1 bis 4, **dadurch gekennzeichnet, daß** die Kerben auf den sich gegenüberliegenden Seiten zueinander "auf Lücke" versetzt angeordnet sind.

6. Trägerkatalysatoren nach Anspruch 1 bis 5, **dadurch gekennzeichnet, daß** die Kerben halbrund, rechteckig, trapezförmig oder V-förmig sind.

7. Trägerkatalysatoren nach Anspruch 1 bis 6, **dadurch gekennzeichnet, daß** die Kerbenbreite größer als die Wandstärke des Hohlzylinders ist.

8. Trägerkatalysatoren nach Anspruch 1 bis 7, **dadurch gekennzeichnet, daß** die Kerbenbreite kleiner als die Wandstärke des Hohlzylinders ist.

9. Trägerkatalysatoren nach Anspruch 1 bis 8, **dadurch gekennzeichnet, daß** die Kerbenbreite so gewählt wird, daß sich die Katalysatorträger untereinander nicht verhaken können.

10. Trägerkatalysatoren nach Anspruch 1 bis 9 **dadurch gekennzeichnet, daß** die Hohlzylinder aus Steatit, Siliciumcarbid, Steingut, Porzellan, Siliciumdioxid, Silicate, Aluminiumoxid, Aluminate oder aus Mischungen dieser Stoffen bestehen.

11. Trägerkatalysatoren nach Anspruch 1 bis 10, **dadurch gekennzeichnet, daß** Hohlzylinder mit einer Höhe von 4 bis 10 mm, einem Außendurchmesser von 6 bis 10 mm und einer Wandstärke von 1 bis 2 mm eingesetzt werden.

12. Verwendung von Trägerkatalysatoren nach Anspruch 1 bis 11 bei der Gasphasenoxidation von Kohlenwasserstoffen.

13. Verwendung nach Anspruch 12 bei der Gasphasenoxidation von o-Xylol und/oder Naphthalin zu Phthalsäureanhydrid.

14. Verwendung nach Anspruch 12 bei der Gasphasenoxidation von gesättigten und/oder ungesättigten Kohlenwasserstoffen zu Essigsäure.

## Claims

1. Supported catalyst consisting of an active material on inert supports in the shape of hollow cylinders, **characterized in that** the hollow cylinders have one or more notches in the upper and/or lower flat side of the hollow-cylinder walls, so that the interior of the hollow cylinders is connected, via openings, to the spaces surrounding the hollow cylinders.

2. Supported catalysts according to Claim 1, **characterized in that** the number of notches on a flat side of the hollow cylinder is between 2 and 8.

3. Supported catalysts according to either of Claims 1 and 2, **characterized in that** the notches are regularly distributed.

4. Supported catalysts according to any of Claims 1 to 3, **characterized in that** the notches are irregularly distributed.

5. Supported catalysts according to any of Claims 1 to 4, **characterized in that** the notches on the opposite sides are arranged offset relative to one another "in gaps".

6. Supported catalysts according to any of Claims 1 to 5, **characterized in that** the notches are semicircular, rectangular, trapezoidal or V-shaped.

7. Supported catalysts according to any of Claims 1 to 6, **characterized in that** the notch width is greater than the wall thickness of the hollow cylinder.

8. Supported catalysts according to any of Claims 1 to 7, **characterized in that** the notch width is smaller than the wall thickness of the hollow cylinder.

9. Supported catalysts according to any of Claims 1 to 8, **characterized in that** the notch width is chosen so that the catalyst supports cannot interlock with one another.

10. Supported catalysts according to any of Claims 1 to 9, **characterized in that** the hollow cylinders consist of steatite, silicon carbide, earthenware, porcelain, silica, silicates, alumina, aluminates or mixtures of these substances.

11. Supported catalysts according to any of Claims 1 to 10, **characterized in that** hollow cylinders having a height of from 4 to 10 mm, an external diameter of from 6 to 10 mm and a wall thickness of from 1 to 2 mm are used.

12. Use of supported catalysts according to any of Claim 1 to 11 in the gas-phase oxidation of hydrocarbons.

13. Use according to Claim 12 in the gas-phase oxidation of oxylene and/or naphthalene to phthalic anhydride.

14. Use according to Claim 12 in the gas-phase oxidation of saturated and/or unsaturated hydrocarbons to acetic acid.

## Revendications

1. Catalyseurs supportés constitués d'une masse active sur des corps support inertes sous forme de cylindres creux, **caractérisés en ce que** les cylindres creux présentent une ou plusieurs nervures sur le côté plat supérieur et/ou inférieur des parois des cylindres creux, de telle manière que l'espace intérieur des cylindres creux est relié avec les espaces entourant les cylindres creux via des ouvertures.

2. Catalyseurs supportés selon la revendication 1, **caractérisés en ce que** le nombre de nervures sur un côté plat du cylindre creux est compris entre 2 et 8.

3. Catalyseurs supportés selon les revendications 1 à 2, **caractérisés en ce que** les nervures sont réparties régulièrement.

4. Catalyseurs supportés selon les revendications 1 à 3, **caractérisés en ce que** les nervures sont réparties irrégulièrement.

5. Catalyseurs supportés selon les revendications 1 à 4, **caractérisés en ce que** les nervures sont disposées sur les faces opposées de manière décalée par rapport à des "vides" les unes par rapport aux autres.

6. Catalyseurs supportés selon les revendications 1 à 5, **caractérisés en ce que** les nervures sont en forme de demi-cercle, de rectangle, de trapèze ou de V.

7. Catalyseurs supportés selon les revendications 1 à 6, **caractérisés en ce que** la largeur des nervures est supérieure à l'épaisseur de paroi du cylindre creux.

8. Catalyseurs supportés selon les revendications 1 à 7, **caractérisés en ce que** la largeur des nervures est inférieure à l'épaisseur de la paroi du cylindre creux.

9. Catalyseurs supportés selon les revendications 1 à 8, **caractérisés en ce que** la largeur des nervures est choisie de telle manière que les supports des catalyseurs ne peuvent pas s'accrocher entre eux.

10. Catalyseurs supportés selon les revendications 1 à 9, **caractérisés en ce que** les cylindres creux sont constitués de stéatite, de carbure de silicium, de grès, de porcelaine, de dioxyde de silicium, de silicates, d'oxyde d'aluminium, d'aluminates ou de mélanges de ces substances.

11. Catalyseurs supportés selon les revendications 1 à 10, **caractérisés en ce qu'**on utilise des cylindres creux présentant une hauteur de 4 à 10 mm, un diamètre extérieur de 6 à 10 mm et une épaisseur de paroi de 1 à 2 mm.

12. Utilisation de catalyseurs supportés selon les revendications 1 à 11 dans l'oxydation en phase gazeuse d'hydrocarbures.

13. Utilisation selon la revendication 12 lors de l'oxydation en phase gazeuse de o-xylène et/ou de naphtalène en anhydride de l'acide phtalique.

14. Utilisation selon la revendication 12 lors de l'oxydation en phase gazeuse d'hydrocarbures saturés et/ou insaturés en acide acétique.
